# FASCICULE DE BREVET EUROPEEN

(11) **EP 1 013 223 B1**
(45) Date de publication et mention de la délivrance du brevet: **15.04.2009**
(21) Numéro de dépôt: 99403246.4
(22) Date de dépôt: 22.12.1999
(51) Int. Cl.: A61B 5/20, A61B 5/03

(54) **Sonde urétrale pour examen urodynamique**
Harnröhrensonde für urodynamische Untersuchungen
Urethral probe for urodynamic examinations

(30) Priorité: 23.12.1998 FR 9816373
(43) Date de publication de la demande: 28.06.2000
(73) Titulaire: Peters Surgical, 93000 Bobigny (FR)
(72) Inventeur: Cannon, Robert Lee III, 53700 St. Germain de Coulamer (FR); Lourdel, Philippe, 95000 Cergy-Pontoise (FR)
(74) Mandataire: Texier, Christian

(56) Documents cités:
- EP-A- 0 878 166
- US-A- 4 538 621
- US-A- 5 385 563
- L. LE NORMAND ET AL.: "Fiabilité des mesures de pressions obtenues par les sondes utilisées pour la réalisation du profile de pression uréthral par la méthode perfusionelle" PROGRÈS EN UROLOGIE,1995, pages 980-984, XP002114407

## Description

L'invention se rapporte au domaine médical des examens urologiques du type urodynamique. Elle vise en particulier une sonde permettant de pratiquer cet examen.

L'examen urodynamique a pour objet d'étudier le comportement dynamique du système vésico-sphinctérien inclus dans l'enceinte abdominale. On utilise une machine spécialisée qui enregistre les pressions mesurées dans la vessie, l'urètre et l'abdomen.

Dans les systèmes à prise de pression à eau, on dispose d'une sonde qui mesure les pressions vésicales et urétrales et une sonde pour la prise de pression abdominale.

Le principe de mesure de la pression vésicale consiste à remplir la vessie avec de l'eau et à mesurer la pression intravésicale.

La prise de pression urétrale consiste à perfuser un liquide à débit constant au travers d'un orifice latéral d'une sonde introduite dans l'urètre. On mesure ensuite les variations de pression en amont en fonction de la position de la sonde le long de l'urètre.

La demande de brevet FR 97 06003 déposée le 15/05/1997 par la demanderesse décrit un dispositif amélioré pour pratiquer ce type d' examen.

Une mesure particulière que l'on pratique en urologie concerne le coefficient de transmission. Elle consiste à enregistrer en même temps la pression dans la vessie et dans l'urètre pendant des efforts répétés de toux ou avec percussion sur l'abdomen. Dans l'urètre on mesure notamment la pression au niveau ou juste dessous le sphincter urinaire. Les courbes de pression en fonction du temps tracées à partir de ces mesures montrent un pic synchrone et sensiblement de même amplitude pour la vessie et l'urètre. Le coefficient de transmission désigne le rapport d'amplitude des pics de pression dans l'urètre et dans la vessie. Ainsi pour un patient sans trouble particulier, la transmission est bonne et le coefficient mesuré est alors proche de 1. En cas d'insuffisance urétrale on constate que la courbe de pression vésicale est supérieure à celle de la pression urétrale pendant la toux. Si c'est le contraire le patient est susceptible d'avoir des problèmes neurologiques et le résultat doit être vérifié par des contrôles complémentaires.

Avec les dispositifs de l'art antérieur, on a constaté que les mesures n'étaient pas fiables. Les expériences cliniques montrent que les tests donnent des résultats dépendant largement de l'assortiment des éléments de mesure utilisés : sondes, connecteurs, tubes prolongateurs ou capteurs. Il est même apparu que pour certaines combinaisons les résultats étaient indépendants du patient.

L'invention a pour objet la réalisation d'un dispositif de mesure urodynamique, destiné notamment à la mesure du profil urétral et du coefficient de transmission, autorisant un diagnostic fiable indépendamment des éléments le constituant.

L'invention a encore pour objet un dispositif de mesure urodynamique comprenant une sonde urétrale et un prolongateur de sonde et autorisant un diagnostic fiable.

Conformément à l'invention on parvient à ces objectifs au moyen d'une sonde urétrale conforme à la revendication 1 annexée, un prolongateur conforme à la revendication 6 annexée et un dispositif de mesure urodynamique conforme à la revendication 8 annexée.

L'invention sera mieux comprise à la lecture de la description qui suit d'un mode de réalisation en relation avec les dessins annexés sur lesquels :
- la figure 1 représente schématiquement un montage permettant de réaliser une analyse urodynamique ;
- la figure 2 représente des courbes de transmission de pression caractéristiques de sondes de l'art antérieur ;
- la figure 3 représente des courbes de transmission de pression pour un couple de canules rigides non équilibrées ;
- la figure 4 représente des courbes de transmission de pression pour un couple de canules rigides équilibrées ;
- la figure 5 représente des courbes de transmission de pression avec palier pour un couple de canules non équilibrées ;
- la figure 6 représente des courbes de transmission de pression pour une sonde conforme à l'invention. Elle est équilibrée ;
- la figure 7 représente des courbes de transmission de pression pour une sonde équilibrée équipée d'un prolongateur de l'art antérieur. l'ensemble est déséquilibré.

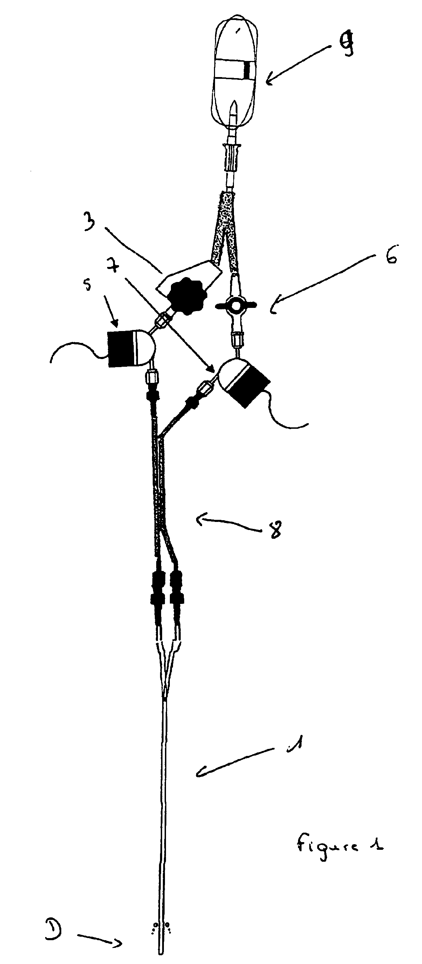

On a représenté sur la figure 1 un montage permettant de réaliser des mesures urodynamiques. La sonde 1 réalisée en un matériau relativement rigide, par exemple du PVC, comprend deux canaux ou lumières : un canal vésical et un canal urétral. Le canal vésical débouche à l'extrémité distale D de la sonde et le canal urétral débouche sur la paroi de la sonde un peu en retrait de son extrémité distale. De l'autre côté, les deux canaux prolongés par un tube prolongateur 8 se terminent par des moyens de connexion qui permettent de brancher chaque canal à une source de liquide et à un capteur de pression (5, 7). Ceux-ci sont reliés électriquement à un appareil enregistreur non représenté.

Une méthode de mesure du coefficient de transmission comprend les étapes suivantes :
On introduit la sonde par son extrémité distale dans l'urètre du patient et on la positionne de façon que le canal vésical débouche dans la vessie et que le canal urétral soit au niveau du sphincter urinaire ou juste en dessous.
On remplit progressivement la vessie.
Lorsqu'elle est partiellement pleine, on perfuse le canal urétral à un débit déterminé compris entre 1 et 5 ml par minute.
On fait alors tousser le patient, et on enregistre en même temps la pression dans la vessie et la pression à l'intérieur du canal urétral. Cette dernière est fonction de la force de fermeture de l'orifice distal du canal exercée par la paroi de l'urètre.
Les capteurs de pression sont disposés à l'extrémité opposée, proximale, des deux canaux; ils enregistrent en fait les pressions régnant en cet endroit des canaux. Jusqu'à présent, en enregistrant les pressions régnant au niveau des capteurs, on considérait que l'on obtenait une image exacte des pressions régnant à l'extrémité distale de la sonde sans réaliser qu'elles étaient altérées différemment lors de leur transmission le long des canaux. En outre, on a constaté que les pressions effectivement enregistrées dépendaient de la nature des canaux entre les extrémités distales et proximales.

Pour illustrer l'influence de la nature des canaux sur la fiabilité des mesures, on a procédé à l'expérience suivante recréant les conditions de mesure clinique :
On plonge l'extrémité distale de la sonde dans une enceinte contenant de l'eau. Le canal vésical est alors rempli d'eau et le canal urétral communique également avec l'enceinte
On perfuse le canal urétral à un débit de 2 ml/min. par exemple.
On crée une impulsion de pression à l'intérieur du récipient et on enregistre la pression de l'eau régnant à l'extrémité proximale de chacun des canaux, au moyen de capteurs disposés à leur niveau.

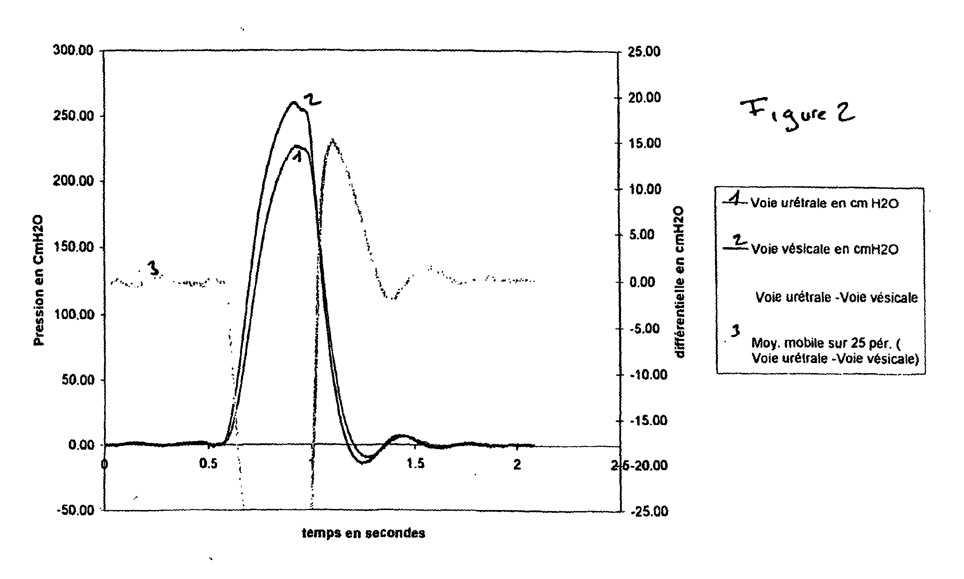

On a réalisé cette simulation sur une sonde de l'art antérieur, et on a relevé les valeurs des pressions en fonction du temps. Les courbes de la figure 2 reproduisent les résultats obtenus : variation des pressions dans le temps pour les deux canaux (échelle de gauche en cm de colonne d'eau) et variation de leur différence dans le temps (échelle de droite en cm de colonne d'eau). On constate, au niveau des pics de pression, un écart important entre les deux canaux. Ainsi il apparaît qu'avec une sonde de l'art antérieur on mesure un différentiel de pression créé par le dispositif de mesure. On constate par ailleurs que lorsque l'on crée une augmentation de pression statique, il y a également une différence entre les canaux. Les résultats de mesures in vivo que l'on peut réaliser avec ce type de sonde comportent donc un facteur d'incertitude important.

Conformément à l'invention, on détermine les diamètres et les longueurs des deux canaux de telle façon que la pression différentielle entre les canaux au niveau des capteurs de pression soit sensiblement la même qu'au niveau de l'extrémité distale de la sonde. L'invention a donc consisté à « équilibrer » les deux voies en sélectionnant pour un matériau donné et les conditions de fonctionnement également données, c'est à dire la pression du liquide dans le canal vésical et le débit de la voie urétrale, les diamètres et les longueurs que doivent présenter les deux canaux afin que l'écart des pressions à l'autre extrémité soit aussi faible que possible. Un écart de 5 % est satisfaisant.

La détermination des dimensions et des matériaux pour un tel dispositif de mesure des pressions cliniques comprenant la sonde, les prolongateurs le cas échéant, les moyens de connexion et les capteurs devrait se ramener à un simple problème de dynamique des fluides. Malheureusement dans la réalité ce problème ne peut être résolu par le calcul. Les cathéters ont souvent des voies dont le diamètre n'est ni circulaire ni constante sur toute leur longueur. Ils ont également, ainsi que les tubes prolongateurs, une compliance non linéaire et non constante. '

Un moyen pour déterminer les paramètres géométriques est de procéder expérimentalement par approximations successives à partir de canules de différents diamètres et longueurs.

On décrit ci-après une méthode de mise au point d'une sonde.

On utilise des canules rigides de section circulaire connue. Elles ne se dilatent pas dans la plage des pressions à mesurer. On plonge l'extrémité de la canule dans une enceinte de mise sous pression. Ensuite on enregistre les différences de transmission en dynamique entre un capteur relié à la canule et un capteur directement connecté à cette enceinte. On réalise ces essais avec des canules présentant des diamètres différents. On sélectionne celles qui donnent une transmission satisfaisante pour une longueur donnée de canule (correspondant à la longueur du canal de mesure de la sonde définie) et pour des débits de perfusion donnés : 0 ml. pour le canal vésical 1 et 2 ml/min. pour le canal urétral. On définit ainsi des couples de canules simulant les voies de prise de pression de la sonde équilibrée à réaliser.

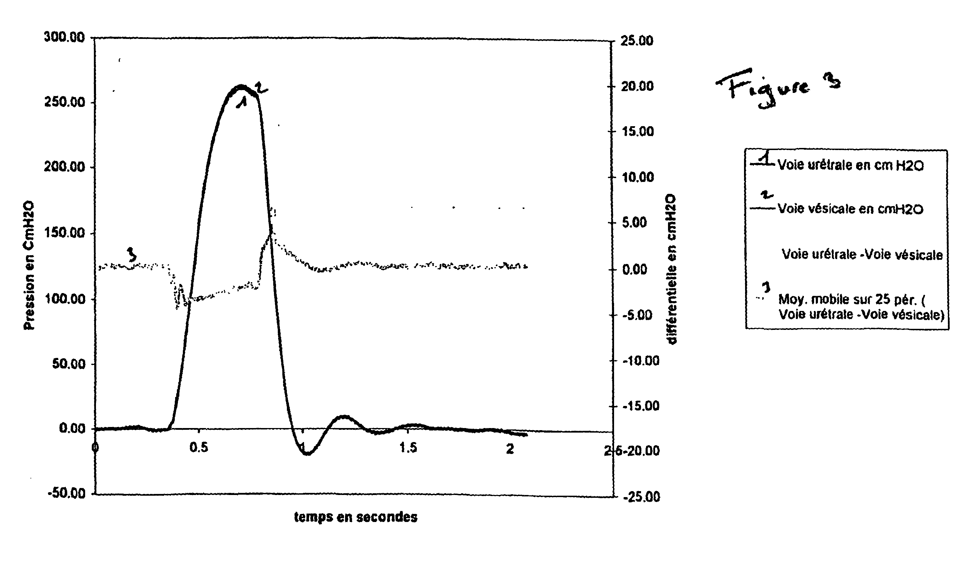

Par exemple, les courbes de la figure 3 illustrent le comportement d'un couple de canules rigides de diamètre intérieur 0,74 mm, lorsque l'une est perfusée et l'autre non. On observe que le signal de pression mesuré n'est pas le même dans les deux cas. Une sonde présentant deux canaux rigides de cette dimension ne serait pas équilibrée.

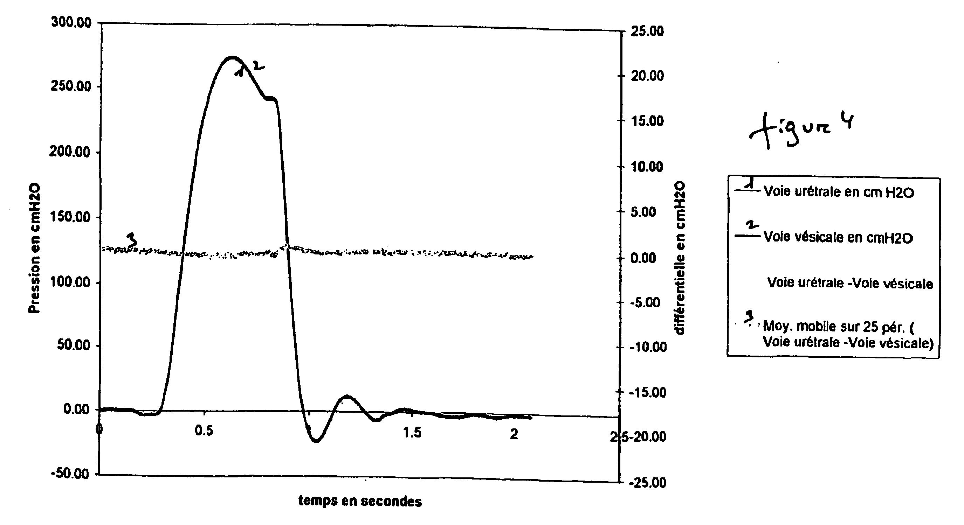

La figure 4 montre les mesures effectuées sur un couple de canules métalliques selon les caractéristiques suivantes :

| | Diamètre | longueur | perfusée |
|---|---|---|---|
| Canule 1(urétrale) | 1.5 mm | 34 cm | 2 ml/min. |
| Canule 2 (vésicale) | 1.25 mm | 40 cm | non |

On constate que la courbe différentielle reste à zéro dans le temps. La réponse à l'impulsion de pression est donc la même dans les deux canules. On peut donc théoriquement réaliser une sonde présentant ces caractéristiques et obtenir des résultats de tests cliniques fiables.

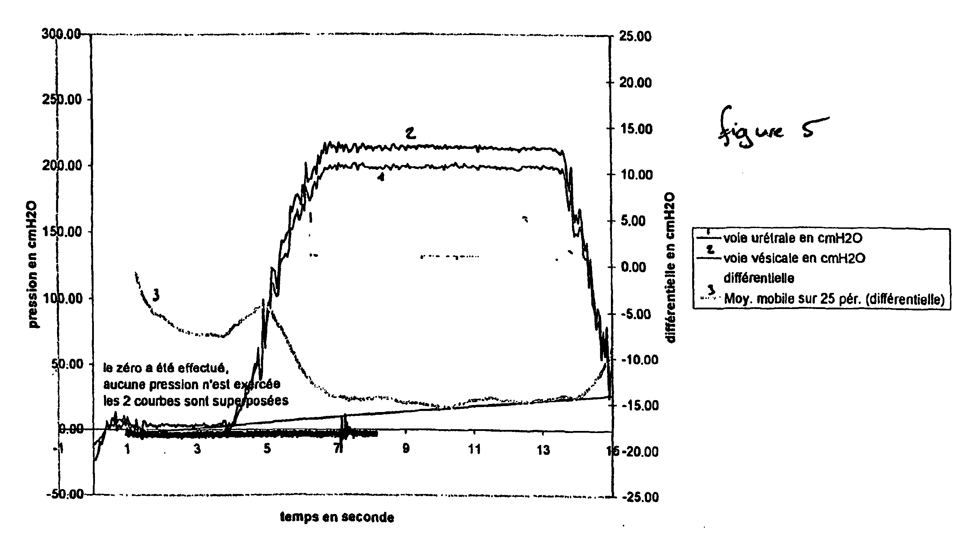

On réalise ensuite des mesures de pression statique par élévation de la pression de l'enceinte en palier. On vérifie ainsi quels couples de canules permettent d'obtenir une pression identique sur chacune des voies à chacun de ces paliers parmi les couples précédemment sélectionnés et ce, pour les mêmes débits. La figure 5 montre un exemple d'enregistrement avec palier de pression pour une sonde non équilibrées de l'art antérieur. Enfin en tenant compte des données recueillies dans les étapes précédentes on peut déterminer les formes, les sections les matériaux et les épaisseurs de paroi des tubes nécessaires à la réalisation de sondes.

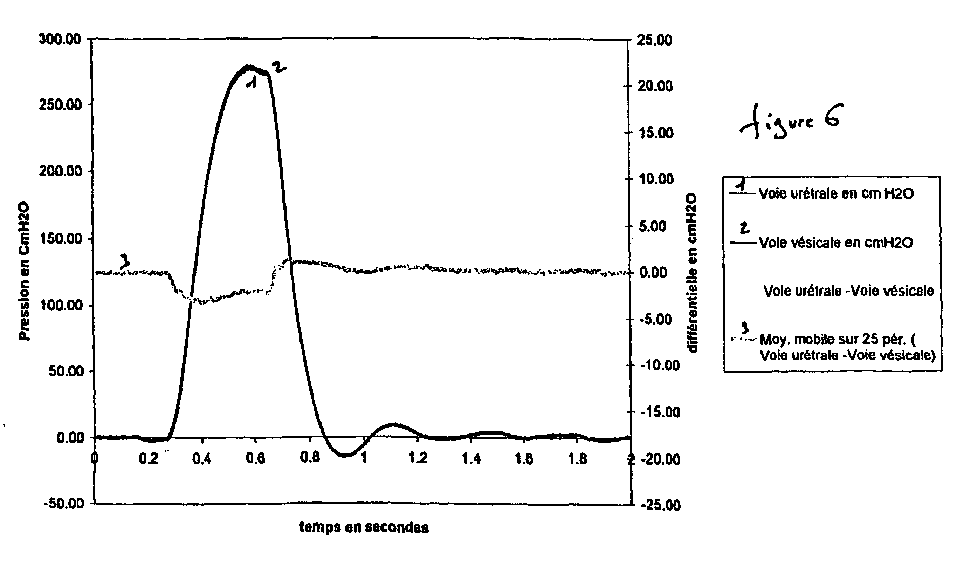

La figure 6 montre les résultats des mesures effectuées sur une sonde « équilibrée » réalisée à partir de ces indications. Il s'agit d'une sonde CH10 en PVC à 2 voies concentriques réalisée avec un tube de diamètres (2,30 mm, 3,30 mm) et un tube interne de diamètres (0,90 mm, 1,40 mm). La voie centrale sert de prise de pression vesicale non perfusée et la voie périphérique sert de prise de pression urétrale perfusée.

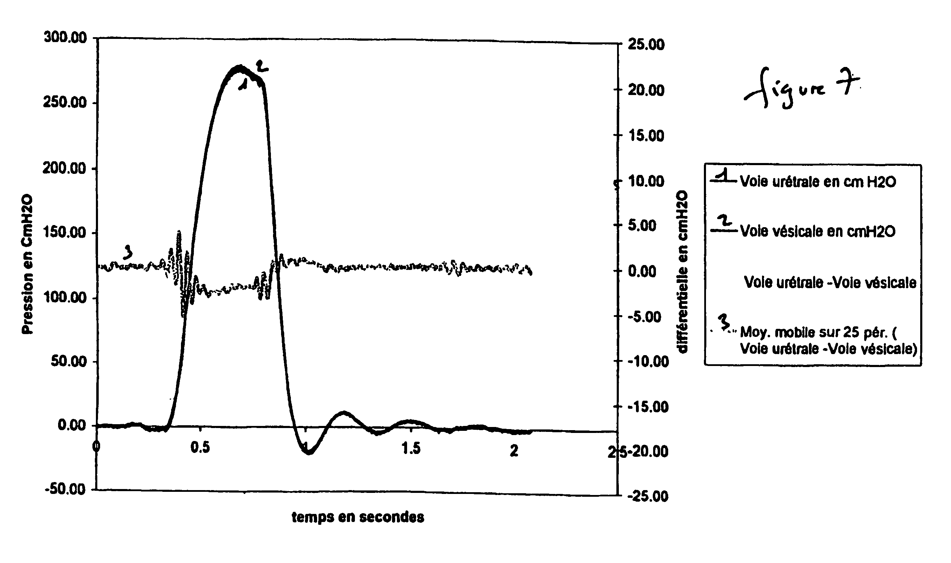

Dans la mesure où les sondes sont utilisées fréquemment avec des tubes prolongateurs, il est nécessaire également de réaliser des prolongateurs qui ne vont déséquilibrer la sonde avec laquelle on effectue les mesures. La figure 7 montre qu'en équipant une sonde équilibrée avec un prolongateur de l'art antérieur à savoir de longueur 1 m et de diamètres 1,25 et 1,33 mm pour les deux tubes; l'ensemble est déséquilibré. Conformément à l'invention, on réalise donc des prolongateurs qui ne déséquilibrent les sondes. Leur méthode de mise au point est la même que pour la sonde. On obtient ainsi un prolongateur avec une voie perforée de 2,8 mm et une voie non perforée de diamètre égal à 1,25 mm.

## Revendications

1. Sonde urétrale (1) comprenant un canal vésical et un canal urétral pour examen urodynamique avec une extrémité distale (D) destinée à être introduite dans l'urètre du patient et une extrémité proximale pour connexion de chaque canal à une source de liquide (9) et à un moyen (5, 7) capteur de pression **caractérisée en ce que** la section et la longueur de chacun des deux canaux sont déterminées de telle façon que lorsqu'on relie le canal vésical à une source de liquide statique, que l'on perfuse le canal urétral et que l'on crée une augmentation de pression en même temps à l'extrémité distale de chacun des deux canaux, l'écart entre les pressions des deux canaux mesurées au niveau des capteurs de pression est inférieur à 5%.

2. Sonde urétrale (1) selon la revendication précédente **caractérisée en ce que** l'augmentation de pression est comprise entre 0 et 300cm H₂0.

3. Sonde urétrale (1) selon l'une des revendications précédentes **caractérisée en ce que** le canal urétral est perfusé à un débit compris entre 1 et 5ml par minute.

4. Sonde urétrale (1) selon l'une des revendications précédentes **caractérisée en ce que** les deux canaux sont concentriques.

5. Sonde urétrale (1) selon l'une des revendications 1 à 3 **caractérisée en ce qu'**elle comprend deux ou trois canaux non concentriques.

6. Prolongateur (8) de sonde urétrale selon l'une des revendications précédentes, comprenant un premier tube muni de moyens de connexion avec l'extrémité proximale du canal vésical et un deuxième tube muni de moyens de connexion avec l'extrémité proximale du canal urétral, afin de permettre son raccordement entre l'extrémité proximale de la sonde (1) et les capteurs de pression (5, 7) d'autre part,
**caractérisé en ce que** le diamètre et la longueur de chacun des deux tubes sont déterminées de telle façon que, lorsqu'ils sont raccordés aux canaux de la sonde (1), et lorsqu'on relie le premier tube à une source de liquide statique, que l'on perfuse le canal urétral par l'intermédiaire du deuxième tube, et que l'on crée une augmentation de pression en même temps à l'extrémité distale de chacun des deux canaux, l'écart entre les pressions des deux tubes mesurées au niveau des capteurs de pression est inférieur à 5%.

7. Prolongateur (8) de sonde urétrale selon la revendication précédente, **caractérisé en ce qu'**il est constitué de deux tubes solidaires l'un de l'autre sur la majeure partie de leur longueur.

8. Dispositif de mesure urodynamique comprenant une sonde urétrale (1) et un prolongateur (8) de sonde urétrale, la sonde urétrale (1) comprenant un canal vésical et un canal urétral pour examen urodynamique avec une extrémité distale (D) destinée à être introduite dans l'urètre du patient et une extrémité proximale, le prolongateur (8) comprenant un premier tube muni de moyens de connexion avec l'extrémité proximale du canal vésical, et un deuxième tube muni de moyens de connexion avec l'extrémité proximale du canal urétral, afin de permettre son raccordement entre l'extrémité proximale de la sonde (1) d'une part et des capteurs de pression (5, 7) d'autre part,
un canal vésical prolongé étant ainsi formé du canal vésical et du premier tube,
un canal urétral prolongé étant ainsi formé du canal urétral et du deuxième tube,
**caractérisé en ce que** la section et la longueur de chacun des deux canaux prolongés sont déterminées de telle façon que lorsqu'on relie le canal vésical prolongé à une source de liquide statique, que l'on perfuse le canal urétral prolongé et que l'on crée une augmentation de pression en même temps à l'extrémité distale du canal urétral et du canal vésical, l'écart entre les pressions des deux canaux prolongés mesurées au niveau des capteurs de pression est inférieur à 5%.

## Claims

1. An urethral probe (1) comprising a vesical duct and an urethral duct for urodynamic examination with a distal end (D) intended to be introduced into the urethra of the patient and a proximal end for connection of each duct to a source of liquid (9) and to a pressure sensing means (5,7), **characterized in that** the section and the length of each of both ducts are determined so that when the vesical duct is connected to a static liquid source, that the urethral duct is perfused and an increase in pressure is created at the same time at the distal end of each of both ducts, the difference between the pressures of both ducts as measured at the pressure sensors is less than 5%.

2. The urethral probe (1) according to the preceding claim, **characterized in that** the increase in pressure is comprised between 0 and 300 cm H₂O.

3. The urethral probe (1) according to any of the preceding claims, **characterized in that** the urethral duct is perfused at a flow rate comprised between 1 and 5 ml per minute.

4. The urethral probe (1) according to any of the preceding claims, **characterized in that** both ducts are concentric.

5. The urethral probe (1) according to any of claims 1 to 3, **characterized in that** it comprises two or three non-concentric ducts.

6. An extender (8) of an urethral probe according to any of the preceding claims, comprising a first tube provided with means for connection with the proximal end of the vesical duct and a second tube provided with means for connection with the proximal end of the urethral duct, in order to allow it to be connected between the proximal end of the probe (1) on the one hand and the pressure sensors (5,7) on the other hand, **characterized in that** the diameter and the length of each of both tubes are determined so that, when they are connected to the ducts of the probe (1) and when the first tube is connected to a static water source, that the urethral duct is perfused via the second tube, that an increase in pressure is created at the same time at the distal end of each of both ducts, the difference between the pressures of both tubes as measured at the pressure sensors is less than 5%.

7. The urethral probe extender (8) according to the preceding claim, **characterized in that** it consists of two tubes integral with each other over the major portion of their length.

8. An urodynamic measurement device comprising an urethral probe (1) and an urethral probe extender (8), the urethral probe (1) comprising a vesical duct and an urethral duct for urodynamic examination with a distal end (D) intended to be introduced into the urethra of the patient and a proximal end,
the extender (8) comprising a first tube provided with means for connection with the proximal end of the vesical duct and a second tube provided with means for connection with the proximal end of the urethral duct, in order to allow it to be connected between the proximal end of the probe (1) on the one hand and pressure sensors (5,7) on the other hand,
an extended vesical duct being thereby formed of the vesical duct and the first tube,
an extended urethral duct being thereby formed of the urethral duct and the second tube,
**characterized in that** the section and the length of each of both extended ducts are determined so that when the extended vesical duct is connected to static liquid source, that the urethral duct is perfused and an increase in pressure is created at the same time at the distal end of the vesical duct and of the urethral duct, the difference between the pressures of both extended ducts as measured at the pressure sensors is less than 5%.

## Patentansprüche

1. Harnröhrensonde (1), einen Blasenkanal und einen Harnröhrenkanal für urodynamische Untersuchungen umfassend, mit einem distalen Ende (D), das dazu bestimmt ist, in die Harnröhre des Patienten eingeführt zu werden, und einem proximalen Ende zur Verbindung jedes Kanals mit einer Flüssigkeitsquelle (9) und einem Mittel (5, 7) zur Druckmessung, **dadurch gekennzeichnet, dass** der Querschnitt und die Länge der jeweiligen zwei Kanäle derart bestimmt werden, dass, wenn der Blasenkanal mit einer statischen Flüssigkeitsquelle verbunden wird, der Harnröhrenkanal perfundiert wird und gleichzeitig am distalen Ende der jeweiligen zwei Kanäle der Druck erhöht, wobei die Differenz zwischen den auf Ebene der Druckmessmittel gemessenen Druckwerte der zwei Kanälen kleiner als 5 % ist.

2. Harnröhrensonde (1) nach vorangehendem Anspruch, **dadurch gekennzeichnet, dass** die Druckerhöhung zwischen 0 und 300 cm H₂O inklusive ist.

3. Harnröhrensonde (1) nach einem der vorangehenden Ansprüche, **dadurch gekennzeichnet, dass** der Harnröhrenkanal mit einem Durchfluss zwischen 1 und 5 ml inklusive pro Minute perfundiert wird.

4. Harnröhrensonde (1) nach einem der vorangehenden Ansprüche, **dadurch gekennzeichnet, dass** die zwei Kanäle konzentrisch sind.

5. Harnröhrensonde (1) nach einem der Ansprüche 1 bis 3, **dadurch gekennzeichnet, dass** sie zwei oder drei nicht konzentrische Kanäle umfasst.

6. Verlängerung (8) für Harnröhrensonde nach einem der vorangehenden Ansprüche, eine erste Röhre umfassend, die mit Verbindungsmitteln mit dem proximalen Ende des Blasenkanals ausgestattet ist, und eine zweite Röhre, die mit Verbindungsmitteln mit dem proximalen Ende des Harnröhrenkanals ausgestattet ist, um ihren Anschluss zwischen dem proximalen Ende der Sonde (1) und den Druckmessmitteln (5, 7) andererseits zu gestatten, **dadurch gekennzeichnet, dass** der Durchmesser und die Länge der jeweiligen zwei Röhren derart bestimmt werden, dass, wenn sie mit den Kanälen der Sonde (1) verbunden sind, und wenn die erste Röhre mit einer statischen Flüssigkeitsquelle verbunden ist, der Harnröhrenkanal über die zweite Röhre perfundiert und gleichzeitig am distalen Ende der jeweiligen zwei Kanäle der Druck erhöht wird, wobei die Differenz zwischen den auf Ebene der Druckmessmittel gemessenen Druckwerte der zwei Röhren kleiner als 5 % ist.

7. Verlängerung (8) für Harnröhrensonde nach vorangehendem Anspruch, **dadurch gekennzeichnet, dass** sie von zwei Röhren gebildet wird, die über den größten Teil ihrer Länge miteinander verbunden sind.

8. Vorrichtung zur urodynamischen Messung, eine Harnröhrensonde (1) und eine Verlängerung (8) für Harnröhrensonde umfassend, wobei die Harnröhrensonde (1) einen Blasenkanal und einen Harnröhrenkanal für urodynamische Untersuchungen umfasst, mit einem distalen Ende (D), das dazu bestimmt ist, in die Harnröhre des Patienten eingeführt zu werden, und einem proximalen Ende,
wobei die Verlängerung (8) eine erste Röhre umfasst, die mit Verbindungsmitteln mit dem proximalen Ende des Blasenkanals ausgestattet ist, und eine zweite Röhre, die mit Verbindungsmitteln mit dem proximalen Ende des Harnröhrenkanals ausgestattet ist, um ihren Anschluss zwischen dem proximalen Ende der Sonde (1) einerseits und den Druckmessmitteln (5, 7) andererseits zu gestatten,
wobei dadurch vom Blasenkanal und der ersten Röhre ein verlängerter Blasenkanal gebildet wird,
wobei dadurch vom Harnröhrenkanal und der zweiten Röhre ein verlängerter Harnröhrenkanal gebildet wird,
**dadurch gekennzeichnet, dass** der Querschnitt und die Länge der jeweiligen zwei verlängerten Kanäle derart bestimmt werden, dass, wenn der verlängerte Blasenkanal mit einer statischen Flüssigkeitsquelle verbunden wird, der verlängerte Harnröhrenkanal perfundiert und gleichzeitig am distalen Ende des Harnröhrenkanals und des Blasenkanals der Druck erhöht wird, wobei die Differenz zwischen den auf Ebene der Druckmessmittel gemessenen Druckwerte der zwei verlängerten Kanälen kleiner als 5 % ist.
